(19) Europäisches Patentamt
European Patent Office
Office européen des brevets

(11) Publication number: **0 003 409**
**B1**

(12) # EUROPEAN PATENT SPECIFICATION

(45) Date of publication of patent specification: **25.11.81**

(51) Int. Cl.³: **C 07 C 99/00,**
C 07 C 101/04 //C07C121/48

(21) Application number: **79300082.9**

(22) Date of filing: **17.01.79**

(54) **Process for producing 4-amino-methylbicyclo(2,2,2)octane-1-carboxylic acid and intermediate compound therefor.**

(30) Priority: **20.01.78 JP 4939/78**
**20.01.78 JP 4940/78**

(43) Date of publication of application:
**08.08.79 Bulletin 79/16**

(45) Publication of the grant of the European patent:
**25.11.81 Bulletin 81/47**

(84) Designated Contracting States:
**BE CH DE FR GB IT LU NL SE**

(73) Proprietor: **DAIICHI SEIYAKU CO. LTD.**
**14-10, Nihonbashi 3-chome**
**Chuoku Tokyo (JP)**

(72) Inventor: **Shimada, Sadakatsu**
**155-32, Kuriyama Yotsukaidomachi**
**Inbagun Chiba (JP)**
Inventor: **Yukimoto, Yusuke**
**50-6, Masago 5-chome Chibashi**
**Chiba (JP)**

(74) Representative: **Crampton, Keith John Allen et al,**
**D YOUNG & CO 10 Staple Inn**
**London WC1V 7RD (GB)**

(56) References cited:
**DE - A - 1 812 979**

**J. ORG. CHEM. vol. 30, No. 5, May 1965 Washington D.C.**
**J. C. KAUER et al.: "Bridgehead-Substituted Bicyclo(2.2.2)Octanes I. Addition of Ethylene to Cyclohexa-1,3-diene-1,4-dicarboxylic Acid Derivatives" pages 1433—1436**

**J. ORG. CHEM. Vol. 32, 11 November 1967**
**F. W. BAKER et al.: "The Preparation of 4-Substituted Bicyclo(2.2.2)Oct-2-Ene-1-Carboxylic Acids" pages 3344—48.**

**J.AM. CHEM. SOC. vol. 75, February 5, 1953 Washington D.C.**
**J. D. ROBERTS et al.: "Synthesis of Some 4-Substituted Bicyclo(2.2.2)Octane-1-Carboxylic Acids" pages 637—639.**

**EP 0 003 409 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for producing 4-amino-methylbicyclo(2,2,2)octane-1-carboxylic acid and intermediate compound therefor

This invention relates to the production of 4-amino-methylbicyclo (2,2,2) octane-1-carboxylic acid, which is a known useful compound having a high anti-plasmin activity.

The conventional process for producing 4-aminomethyl-bicyclo (2,2,2) octane-1-carboxylic acid comprises desulfurizing the bisethylenethioketal of an ester of bicyclo (2,2,2) octa-2,5-dione-1,4-dicarboxylic acid to form a bicyclo (2,2,2) octane-1,4-dicarboxylic ester, partially hydrolysing this compound, forming the corresponding amide, dehydrating the amide, hydrolysing to form 4-cyanobicyclo (2,2,2) octane-1-carboxylic acid, and reducing the latter (see United States Patent No. 3,634,499, issued January 11, 1972 for further details of this process).

However, this process is not industrially advantageous because it consists of many steps and requires complicated operation, and overall yield of the end product is poor. For example, according to the process disclosed in United States Patent No. 3,634,499, the preparation of the intermediate bicyclo (2,2,2) octane-1,4-dicarboxylic acid ester requires many steps as well as the use of various auxiliary materials such as ethane-1,2-dithiol, which is toxic, Raney nickel, which is used in a large amount, and chlorocarbonic ester, which is expensive. In addition, five more steps are needed for producing the end product from the intermediate ester.

Intensive investigations have been carried out to find processes for producing 4-aminomethylbicyclo (2,2,2) octane-1-carboxylic acid without using the above described disadvantageous intermediate compound. The present invention is based on the discovery that by reacting 4-cyanocyclohexa-1,3-diene-1-carboxylic acid or a lower alkyl ester thereof (Formula I) with ethylene under conditions specified below, a 4-cyanobicyclo (2,2,2) oct-2-en-1-carboxylic acid or a lower alkyl ester thereof (Formula II) is readily obtained. By catalytically reducing and (where the ester is used) hydrolysing the compound of Formula II, the end compound represented by Formula IV can quite easily be obtained as shown below.

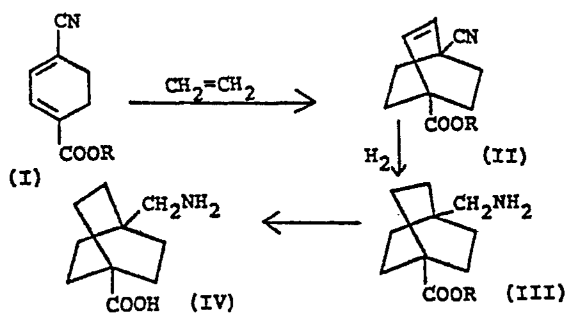


(in each formula, R is hydrogen or a lower alkyl group)

The intermediate compounds, 4-cyanobicyclo (2,2,2) oct-2-en-1-carboxylic acid and its lower alkyl esters, have previously been prepared according to the methods described in J. Org. Chem., *32*, 3344 (1967), but these methods are not satisfactory.

The "lower alkyl" groups mentioned herein are preferably methyl or ethyl but may contain up to 6 carbon atoms.

The first step of the process of this invention involves the reaction for forming the bicyclo (2,2,2) octane ring.

The mechanism of this process is the so-called Diels-Alder Reaction. A similar example to the reaction of the present invention is disclosed in Example 4 of U.S. Patent No. 3,688,005, issued August 29, 1972, in which methyl 4-cyanocyclohexa-1,3-diene-1-carboxylate is reacted with vinyl chloride to produce 1-cyano-4-methoxycarbonyl-(5 or 6)-chlorobicyclo (2,2,2) oct-2-ene. The industrial advantage of this process is not clear insofar as it is difficult to determine the reaction pressure accurately because complicated equipment must be used: moreover, this document makes no mention of the yield of the end product.

In the Diels-Alder reaction, the atmosphere of electrons around the two carbon atoms at the double bond site associated with the reaction should be unstable. Alternatively, these two carbon atoms should be easily chargeable, either positively or negatively, by an electron-attracting or electron-repelling functional substituent linked to these carbon atoms. Such a substituent may be a halogen atom or a cyano group. The best known example of a dienophile is maleic acid anhydride. A symmetrical molecule such as unsubstituted ethylene would therefore be thought to be rather unsuitable because its dienophilic activity would be expected to be lower than that of, say, vinyl chloride, which is used in US Patent No. 3,688,005. Accordingly, to obtain satisfactory results using ethylene as the dienophile would not be expected from consideration of the prior reaction using vinyl chloride. J. Org. Chem., *30*, 1431 (1965) and corresponding US Patent No. 3,081,334, discloses a method of producing a 1,4-disubstituted-bicyclo (2,2,2) oct-2-ene by using ethylene as the dienophile,

but this method requires the use of a special high-pressure reactor since the reaction of 1,3-cyclohexadiene-1,4-dicarboxylate with ethylene to yield dimethyl bicyclo (2,2,2) oct-2-en-1,4-dicarboxylate requires a pressure as high as 1000 kg/cm². Although the US Patent No. 3,081,334 contends that pressures as low as 50 kg/cm² will work, the conversion rates shown at lower pressures are not commercially acceptable.

Under consideration of such conventional techniques, it was believed that the reaction of ethylene with 4-cyanocyclohexa-1,3-diene-1-carboxylic acids would not proceed at all, or would require a very high pressure, or would not produce the end product in a high yield. Surprisingly however, it turned out that the 4-cyanobicyclo (2,2,2) oct-2-ene-1-carboxylic acids would be obtained in a high yield under a pressure very much lower than had been expected.

To be more specific, the pressure required for the reaction involved in the process of the present invention is in the range 60 to 300 kg/cm². The reaction proceeds at a temperature of 100 to 250°C under a pressure within the range defined above and provides a high yield of the end product. A special apparatus is therefore not required but a conventional pressure vessel can be used. The reaction of the present invention may proceed with or without a solvent or catalyst. The purpose of optionally adding a catalyst is to accelerate the rate of the reaction, and suitable catalysts include Lewis acids. Examples of suitable solvents, if used, are aromatic hydrocarbons such as benzene, toluene and xylene, halogenated hydrocarbons such as chloroform and dichloroethane, and hydrocarbons such as hexane and pentane. Hydroquinone or *t*-butylcatechol may also be used to prevent the occurrence of polymerization.

The second step of the process of this invention is a catalytic reduction, in which the compound of Formula II, preferably dissolved in an organic solvent that may or may not contain a small amount of water, such as methanol, ethanol, butanol, ether, dioxane or tetrahydrofuran, is catalytically reduced so as to simultaneously effect the reduction of the cyano group and the reduction of bicyclo(2,2,2)octene to bicyclo(2,2,2)octane, with the result that the compound of Formula II is obtained. It should be noted that it is known separately to reduce the cyano group to aminomethyl and to reduce the vinylene group in the ring structure, in compounds of this type, as disclosed in DE-1,812,979 and US Patent No. 3,688,005 respectively. The catalytic reduction is preferably carried out in a hydrogen atmosphere in the presence of a metal catalyst such as platinum, platinum oxide, ruthenium, Raney nickel, or Raney cobalt. It is sometimes advantageous to use platinum or platinum oxide in the presence of acidic materials such as hydrochloric acid and acetic acid, and also to use ruthenium, Raney nickel or Raney cobalt in the presence of alkaline materials such as ammonia and sodium carbonate.

The third step of the process of this invention comprises hydrolysing the compound (III), when the ester group is present, in the presence of an acid or alkali to provide a salt of the end compound (IV). When the salt is neutralized by a conventional method, for example, using an ion-exchange resin, the compound (IV) is obtained in its free form.

Therefore, according to the process of this invention, the end compound (IV), which it has conventionally required many steps to produce, can be manufactured by fewer steps without using a special reactor of the type that withstands a pressure as high as 1033 kg/cm² (1000 atmospheres).

In this connection, the compound (I) used as the starting material for the process of this invention can be easily prepared by the following method.

4-Cyanocyclohexane-1-carboxylic acid chloride is brominated to form 1,4-dibromo-4-cyanocyclohexane-1-carboxylic acid chloride, esterified with methanol, treated with pyridine to remove the hydrogen bromide so as to provide methyl 4-cyanocyclohexa-1,3-diene-1-carboxylate (boiling point: 101—103°C/1 mmHg).

This invention will now be described in greater detail by reference to the following examples.

Example 1

40.9 g. (0.25 mole) of methyl 4-cyanocyclohexa-1,3-diene-1-carboxylate was dissolved in 40 ml. of benzene and reacted with ethylene at 190—210°C. for 15 hours in a 200-ml autoclave as the ethylene was fed in at 60 kg/cm². After the reaction, the solvent was distilled off the reaction mixture and the residue was recrystallized from methanol to provide 33.5 g. (yield: 70%) of methyl 4-cyanobicyclo(2,2,2)oct-2-en-1-carboxylate having a melting point of 81—83°C.

Example 2

A solution of 28.7 g. (0.15 mole) of the methyl 4-cyano-bicyclo(2,2,2)oct-2-en-1-carboxylate of Example 1 in 300 ml. of 2% HCl/methanol was catalytically hydrogenated in the presence of 0.75 g. of platinum oxide at room temperature until the theoretical amount of hydrogen had been absorbed. After the reaction, the catalyst was filtered off, the filtrate was concentrated under reduced pressure, and the residual solid was recrystallized from mixtures of ethanol and ethyl ether to obtain 32.3 g. (yield: 92%) of methyl 4-aminomethylbicyclo(2,2,2)octane-1-carboxylate hydrochloride having a melting point of 214—217°C.

A solution of 23.4 g (0.1 mole) of the methyl 4-aminomethylbicyclo(2,2,2)octane-1-carboxylate hydrochloride in 100 ml. of 3*N* HCl was hydrolysed by heating for 4 hours under reflux. After the reaction, the solvent was distilled off under reduced pressure, and the residual solid was recrystallized from aqueous ethanol to provide 21.3 g. (yield: 97%) of 4-aminomethylbicyclo(2,2,2)octane-1-carboxylic acid hydrochloride having a melting point of 314—316°C. (measured as a decomposition point in a capillary).

A solution of 17.6 g. (0.08 mole) of the 4-aminomethylbicyclo(2,2,2)octane-1-carboxylic acid hydrochloride in 150 ml. of water was passed through a weak basic ion-exchange resin sold under the trade mark "Amberlite IR-45" (OH type), the eluent was concentrated under reduced pressure, and the residual solid was recrystallized from aqueous ethanol to provide 14.4 g (yield 98%) of 4-aminomethylbicyclo(2,2,2)octane-1-carboxylic acid having a melting point of 271—274°C.

Example 3

A solution of 35.4 g. (0.2 mole) of ethyl 4-cyanocyclohexa-1,3-diene-1-carboxylate in 30 ml. of benzene was reacted with ethylene at 190—210°C. for 15 hours in an autoclave as the ethylene was fed in at 100 kg/cm². The solvent was distilled off the reaction mixture, the residue was dissolved in 200 ml. of ethanol and 36 ml. of concentrated aqueous ammonia, and the solution was catalytically hydrogenated in an autoclave in the presence of 10 ml. of Raney nickel at 50—60 kg/cm². After the reaction, the catalyst was filtered off the reaction mixture, the filtrate was concentrated under reduced pressure, and the residue was dissolved in 200 ml. of 4*N* HCl and heated for 4 hours under reflux. The solvent was then distilled off under reduced pressure, and the residual solid was passed through "Amberlite IR-45" (OH type). The eluate was concentrated under reduced pressure, the residue was recrystallized from aqueous ethanol to provide 23.8 g. (yield: 65%) of 4-aminomethylbicyclo(2,2,2)-octane-1-carboxylic acid.

Example 4

A solution of 29.8 g. (0.2 mole) of 4-cyanocyclohexa-1,3-diene-1-carboxylic acid in 300 ml. of benzene was reacted with ethylene at 200—210°C for 20 hours in an autoclave as the ethylene was fed in at 100 kg/cm². After the reaction, the solvent was distilled off under reduced pressure, and the residue was recrystallized from ethanol to obtain 23.0 g. (yield: 65%) of 4-cyanobicyclo(2,2,2)oct-2-en-1-carboxylic acid having a melting point of 202—204°C.

Example 5

32.6 g (0.2 mole) of methyl 4-cyanocyclohexa-1,3-diene-1-carboxylate was reacted with ethylene at 180—200°C. for 15 hours in a 100-ml autoclave as the ethylene was fed in at 300 kg/cm², with the result that 30.6 g. (yield: 80%) of methyl 4-cyanobicyclo(2,2,2)oct-2-en-1-carboxylate was obtained.

A solution comprising 28.7 g. (0.15 mole) of the methyl 4-cyanobicyclo(2,2,2)oct-2-en-1-carboxylate, 150 ml. of methanol and 27 ml. of concentrated ammonia water was catalytically hydrogenated in an autoclave in the presence of 8 ml. of Raney nickel at 50—60°C. under a hydrogen pressure of 50—60 kg/cm² until the pressure drop stopped. After the reaction, the catalyst was filtered off, the solvent was distilled off, and the residue was dissolved in 150 ml. of 4*N* HCl and hydrolysed by heating for 4 hours under reflux. The solvent was then distilled off under reduced pressure and the residue was recrystallized from aqueous ethanol to provide 29.4 g. (yield: 89.2%) of 4-aminomethylbicyclo(2,2,2)octane-1-carboxylic acid hydrochloride.

Example 6

32.6 g. (0.2 mole) of methyl 4-cyanobicyclo(2,2,2)oct-2-en-1-carboxylate was catalytically hydrogenated in the presence of Raney nickel as the catalyst under the same conditions as used in Example 5. After the reaction, the catalyst was separated from the reaction mixture, the filtrate was dissolved in 130 ml. of 2*N* NaOH and left standing overnight at room temperature. Thereafter the solvent was removed under reduced pressure, the residue was dissolved in 200 ml. of water, the aqueous solution was passed through a column packed with a strong basic ion-exchange resin sold under the trade mark "Diaion SK No. 1" (H type), the column was thoroughly washed with water and eluted with 5% ammonia water, and the fractions of amino acid were concentrated under reduced pressure. The crude amino acid obtained was recrystallized from aqueous ethanol to provide 29.3 g. of 4-aminomethylbicyclo(2,2,2)octane-1-carboxylic acid in a yield of 80%.

**Claims**

1. A process for producing 4-aminomethylbicyclo(2,2,2)octane-1-carboxylic acid or a pharmaceutically acceptable salt thereof which comprises the steps of:

(a) reacting a compound of the formula:

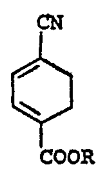

(where R is hydrogen or $C_{1-6}$ alkyl) with ethylene at a pressure in the range 60 to 300 kg/cm$^2$ and a temperature in the range 100 to 250°C to produce a bicyclo(2,2,2)octene of the formula:

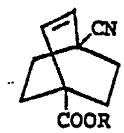

(b) catalytically reducing the said bicyclo(2,2,2)octene and, when R is $C_{1-6}$ alkyl, hydrolysing the reduction product.

2. A process for producing 4-cyanobicyclo(2,2,2)oct-2-en-1-carboxylic acids represented by the formula:

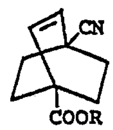

(where R is hydrogen or $C_{1-6}$ alkyl) comprising reacting a compound represented by the formula:

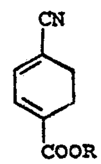

(where R is as defined above) with ethylene at a pressure in the range 60 to 300 kg/cm$^2$ and a temperature in the range 100 to 250°C.

**Revendications**

1. Un procédé de préparation de l'acide 4-aminométhyl-bicyclo(2,2,2)octane-1-carboxylique ou d'un sel de cet acide acceptable pour l'usage pharmaceutique, qui comprend les stades opératoires suivants:

a) on fait réagir un composé de formule

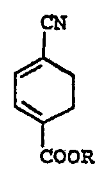

(dans laquelle R représente l'hydrogène ou un groupe alkyle en $C_1$—$C_6$) avec l'éthylène à une pression dans l'intervalle de 60 à 300 kg/cm$^2$ et une température dans l'intervalle de 100 à 250°C, formant ainsi un bicyclo(2,2,2)octène de formule

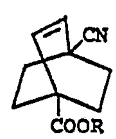

b) on soumet ce bicyclo(2,2,2)octène à réduction catalytique et, lorsque R représente un groupe alkyle en $C_1$—$C_6$, on hydrolyse le produit de réduction.

2. Procédé de préparation des acides 4-cyano-bicyclo(2,2,2)octa-2-ène-1-carboxyliques répondant à la formule

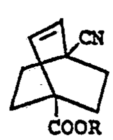

(dans laquelle R représente l'hydrogène ou un groupe alkyle en $C_1$—$C_6$) qui consiste à faire réagir un composé répondant à la formule

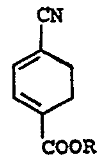

(dans laquelle R a les significations indiquées ci-dessus) avec l'éthylène à une pression dans l'intervalle de 60 à 300 kg/cm$^2$ et à une température dans l'intervalle de 100 à 250°C.

**Patentansprüche**

1. Verfahren zur Herstellung von 4-Aminomethylbicyclo(2,2,2)octan-1-carbonsäure oder eines pharmazeutisch brauchbaren Salzes davon, mit folgenden Stufen:

(a) Reaktion einer Verbindung der Formel:

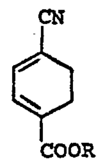

(worin R Wasserstoff oder $C_{1-6}$-Alkyl ist) mit Äthylen bei einem Druck im Bereich von 60 bis 300 kg/cm$^2$ und einer Temperatur im Bereich von 100 bis 250°C zur Bildung eines Bicyclo(2,2,2)octens der Formel:

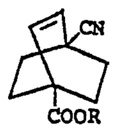

und

(b) katalytische Reduktion dieses Bicyclo(2,2,2)octens und, falls R $C_{1-6}$-Alkyl ist, Hydrolyse des Reduktionsprodukts.

2. Verfahren zur Herstellung von 4-Cyanobicyclo(2,2,2)oct-2-en-1-carbonsäuren, dargestellt durch die Formel:

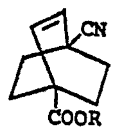

(worin R Wasserstoff oder $C_{1-6}$-Alkyl ist) durch Reaktion einer Verbindung, dargestellt durch die Formel:

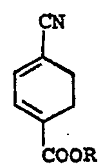

(worin R wie vorstehend definiert ist) mit Äthylen bei einem Druck im Bereich von 60 bis 300 kg/cm$^2$ und einer Temperatur im Bereich von 100 bis 250°C.